# EUROPEAN PATENT SPECIFICATION

(11) **EP 3 875 057 B1**
(45) Date of publication and mention of the grant of the patent: **17.07.2024**
(21) Application number: 20160882.5
(22) Date of filing: 04.03.2020
(51) Int. Cl.: A61F 2/07, A61F 2/95

(54) **CATHETER SYSTEM FOR DELIVERY OF A FILLING BODY TO AN ANEURYSMAL SAC IN A BODY LUMEN**
KATHETERSYSTEM ZUR EINFÜHRUNG EINES FÜLLKÖRPERS IN EINEN ANEURYSMA-SACK IN EINEM KÖRPERLUMEN
SYSTÈME DE CATHÉTER POUR L'ADMINISTRATION D'UN CORPS DE REMPLISSAGE À UN SAC ANÉVRISMAL DANS UNE LUMIÈRE CORPORELLE

(43) Date of publication of application: 08.09.2021
(73) Proprietor: Life Seal Vascular, Inc., Costa Mesa, CA 92626 (US)
(72) Inventor: REIJNEN, Michel Marinus Petrus Johannes, 6824 HL Arnhem (NL)
(74) Representative: 't Jong, Bastiaan Jacob

(56) References cited:
- US-A1- 2009 287 145
- US-A1- 2017 360 993
- US-A1- 2019 192 743

## Description

The invention relates to a catheter system for delivery of a filling body to an aneurysmal sac in a blood vessel, prior or in addition to treatment with an endoprosthesis.

The causes of aneurysms are sometimes unknown but often they are related to atherosclerosis. Aneurysms may occur in any artery in the body, but there is a preference for the aorta, the iliac arteries and the popliteal arteries. Aneurysms may also be iatrogenic or caused by injury. An aneurysm occurs when part of an artery wall weakens, allowing it to balloon out or widen abnormally and can have a fusiform or saccular shape. Such a weakened artery wall can provide a severe health risk for the patient and typically needs to be resolved when a threshold diameter is reached. Other problems, besides the risk on rupture, may include thrombosis and distal embolization.

Typically aneurysms are either treated by surgical bypass or with the endovascular placement of an endoprosthesis, depending on the anatomy of the lesion and patient characteristics. It is known to fill an aneurysm with a thrombogenic wire causing a thrombus to be formed in the aneurysmal sac and strengthening the aneurysm therewith, such that the health risk is reduced.

US 8974487 discloses to introduce a net shaped sac into the aneurysmal sac, filling the net shaped sac with fill members such that the aneurysmal sac is substantially filled, The remaining space can then be filled by thrombi.

These techniques are typically suitable for saccular aneurysms, or pseudoaneurysms, which have a small access opening through the feeding artery. However, a fusiform aneurysm is a widening of the blood vessel with no particular access opening. Filling the space of such a fusiform aneurysm will block the flow through the blood vessel.

Typically a surgical graft, of endovascular placed stentgraft, also named endoprosthesis, is placed in such fusiform aneurysms, which takes away the pressure from the aneurysmal sac. However, leaks may occur, which will again pressurize the aneurysmal sac and could require surgerical or endovascular re-intervention. Such leaks are called endoleaks and occur particularly after endovascular treatment of an aneurysm.

US 5769882 proposes a solution for such fusiform aneurysms. The publication teaches to introduce a tubular element in the blood vessel at the position of the fusiform aneurysm and then inject a liquid sealant between the tubular element and the aneurysmal sac wall. This will reduce the chance on endoleaks.

The sealant could be partially curable. The liquid sealant could pose a problem when the tubular element does not seal sufficiently against the blood vessel wall and liquid sealant could enter the blood stream.

US 2017/360993 relates generally to endovascular prostheses. More particularly, the disclosure relates to endovascular prostheses having an outer surface of a graft material thereof associated with a hydrogel composition, which may swell upon implantation within a blood vessel, thereby mediating various complications associated with endovascular procedures. The hydrogel compositions can also include various stabilizing polymers and active agents to further aid their use in the body.

It is an object of the invention to propose an alternative in which the above mentioned disadvantages are reduced or even removed.

This object is achieved by a catheter system for delivery of a filling body to an aneurysmal sac in a blood vessel, which catheter system comprises:
- a substantially tubular shaft, having a proximal end, a distal end and a guidewire lumen extending between the proximal and distal ends;
- a substantially tubular sheath defining an interior volume, coaxially positioned over the tubular shaft and slidable relative to the tubular shaft;
- a compressible filling body arranged in a compressed state in the interior volume between the sheath and the shaft near the distal end of the tubular shaft;
wherein the compressible filling body is expandable, when released from the sheath, to a porous filling body having a substantially cylindrical lumen and an outer surface having a main dimension perpendicular to the longitudinal axis of the cylindrical lumen, which main dimension is at least 1,5 times the diameter of the cylindrical lumen, characterized in that the expanded filling body is porous and the material of the filling body has an open cell structure, preferably of a shape memory polymer or other memory material.

The main dimension of the outer surface is considered the dimension, which characterized the shape of the cross-section perpendicular to the longitudinal axis of the cylindrical lumen. So, for a cylindrical filling body, this main dimension is the outer diameter. Also, for a sphere shaped outer surface, this main dimension is the diameter of the sphere. For a rectangular cross-section, it would be the largest diagonal and so on.

With the catheter system according to the invention, a porous filling body can be delivered via a blood vessel into the aneurysmal sac of an aneurysm, such that the aneurysmal sac is substantially filled. The substantial cylindrical lumen in the filling body allows for a passthrough opening, in which a conventional stent, stentgraft, graft or other prosthesis can be arranged.

Because the filling body is compressible, the filling can be arranged between the shaft and sheath of the catheter system and can be deployed in the aneurysmal sac by retracting the sheath relative to the shaft, such that the filling body is allowed to expand in flow lumen of the aneurysmal sac.

The porosity of the filling body contributes to the compressibility, but also promotes thrombus forming around the filling body and rapid remodeling of the aneurysm. If any endoleaks do occur, the leaking will be reduced or stopped by the thrombus forming in the aneurysmal sac.

If a type I endoleak occurs despite the arrangement of a stent or the like, the filling body will ensure, that such leakage will only fill the stent, but no longer the aneurysmal sac and re-pressurizing of said aneurysmal sac will be prevented.

Avoidance of type II endoleaks, because the filling body fills the aneurysmal sac, will lead to more shrinkage of the aneurysm, with a positive impact on follow-up regimes and mortality.

In relation to type III endoleaks the filling body will provide lateral stability to the stent or EVAR (Endo Vasculaire Aorta Repair) device and as such there is a lower risk on component separation or material fatigue
Because of the arrangement of the filling body, migration of the EVAR device is very unlikely, as the EVAR device is fitted in the cylindrical lumen of the filling body, such that there is minimal space in lateral directino. This lateral stability will attribute to the proximal stability of the EVAR device. (Distal migration can only occur with an increased angulation of the EVAR device in the sac).

In a preferred embodiment of the catheter system according to the invention, the outer surface is non-cylindrical, preferably bulbous shaped.

In a preferred embodiment of the catheter system according to the invention, a nitinol stent is arranged in the cylindrical lumen of the filling body. A nitinol stent can be compressed and be positioned into the cylindrical lumen, when the filling body is compressed between the shaft and the sheath. When the filling body is deployed into the aneurysmal sac, the nitinol stent will also expand to a designed diameter to ensure a sufficient large cylindrical lumen in the filling body for another stent, stentgraft, graft or prosthesis to be arranged therein.

According to the invention, the material of the filling body has an open cell structure, preferably of a shape memory polymer or another memory material.

The open cell structure provides the porosity of the filling body. Furthermore, the use of a shape memory polymer ensures that the filling body will deploy from the compressed state to a designed expanded state, which will sufficiently fill the aneurysmal sac without causing too much stress onto the wall of the aneurysmal sac.

Even more preferred is a catheter system according to the invention, wherein the material of the filling body is a non-isotropic 3D printed foam with a structure similar to cancellous and cortical fibres. Such a structure allows for good compressibility and allows for good thrombogenic properties when expanded into the aneurysmal sac.

In an example not according to the invention the filling body is composed out of a plurality of thrombogenic wires arranged to a body core in which the substantially cylindrical lumen is provided.

In still a further embodiment of the catheter system according to the invention the diameter of the circumscribing circle of the non-cylindrical outer surface in a plane perpendicular to the longitudinal axis of the substantially cylindrical lumen is between 5cm and 10cm.

The bulbous shape of the outer surface of the filling body should more or less correspond to the lumen of the aneurysmal sac in order to properly fill the aneurysmal sac when expanded. By ensuring that the circumscribing circle of the non-cylindrical outer surface in a plane perpendicular to the longitudinal axis of the substantially cylindrical lumen is between 5cm and 10cm, the filling body would be suitable for most abdominal aneurysms. For aneurysm in other parts of the body, such as the thoracic aorta, the iliac artery and the popliteal artery, other dimensions would be required (2 cm to 10 cm).

In still a further preferred embodiment of the catheter system according to the invention the filling body is coated with a hemostatic coating, such as fibrinogen, fibrin or collagen. The hemostatic coating further promotes thrombus forming around the porous structure of the filling body.

These and other features of the invention will be elucidated in conjunction with the accompanying drawings.
Figure 1 shows a cross-sectional view of an embodiment of the catheter system according to the invention in a first state.
Figure 2 shows the catheter system of figure 2 in a second state.
Figure 3 shows a perspective view of the filling body of the catheter system according to figure 1.
Figure 4 shows a cross-sectional view of the filling body of figure 3.
Figures 5A - 5D show different steps of a method for deploying the catheter system of figure 1.

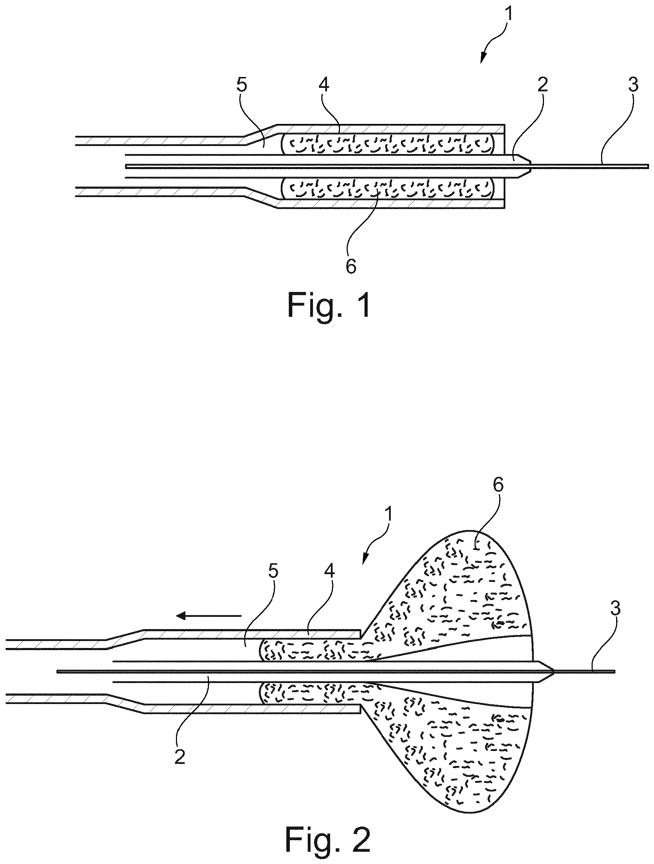

Figure 1 shows a cross-sectional view of a catheter system 1 according to the invention. The catheter system 1 has a tubular shaft 2, through which a guide wire 3 extends. A tubular sheath 4 defining an interior volume 5 is coaxially positioned over the tubular shaft 2.

A compressible filling body 6 is arranged in a compressed state in the interior volume 5 near the distal end of the tubular shaft 2.

Figure 2 shows the catheter system 1 in a partially deployed state. The sheath 4 is retracted relative to the shaft 2, such that the compressible filling body 6 is partially pushed out of the interior volume 5, such that the filling body 6 is allowed to expand.

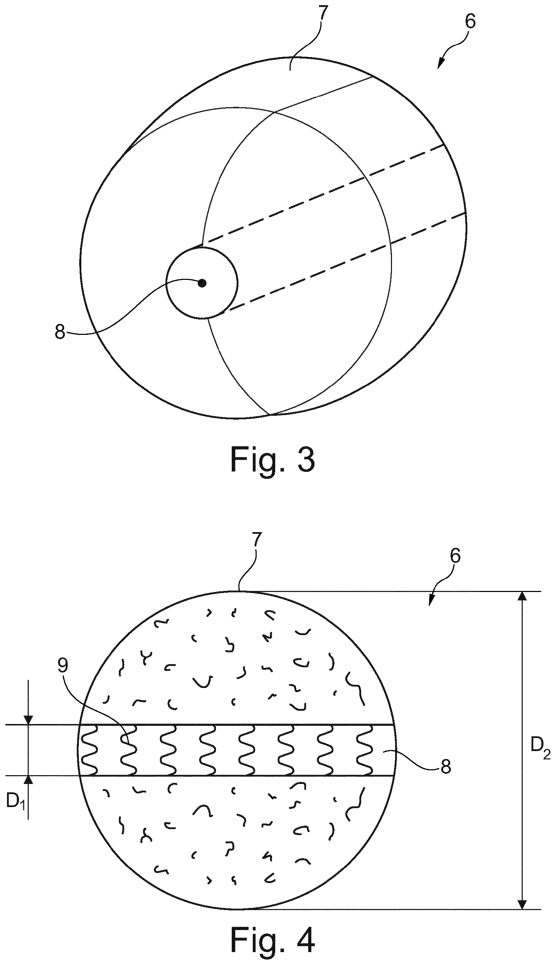

Figure 3 shows the filling body 6 in fully expanded state. The filling body 6 has a bulbous outer surface 7 with a cylindrical lumen 8 extending through the filling body 6.

Figure 4 shows a cross-sectional view of the filling body 6. A nitinol stent 9 is arranged in the cylindrical lumen 8, which has a diameter of D₁. This nitinol stent 9 expands together with the porous filing body when the filling body 6 is moved out of the internal volume 5. The filling body 6 has a bulbous shape and a main dimension of D₂, which is at least 1,5 times larger than the diameter D₁.

Instead of a bulbous shape, the filling body could also have a more cylindrical or even fully cylindrical shape. Relevant is that there is sufficient material available between the cylindrical lumen and the outer surface, in which porous material thrombus forming can occur in order to further fill the aneurysmal sac.

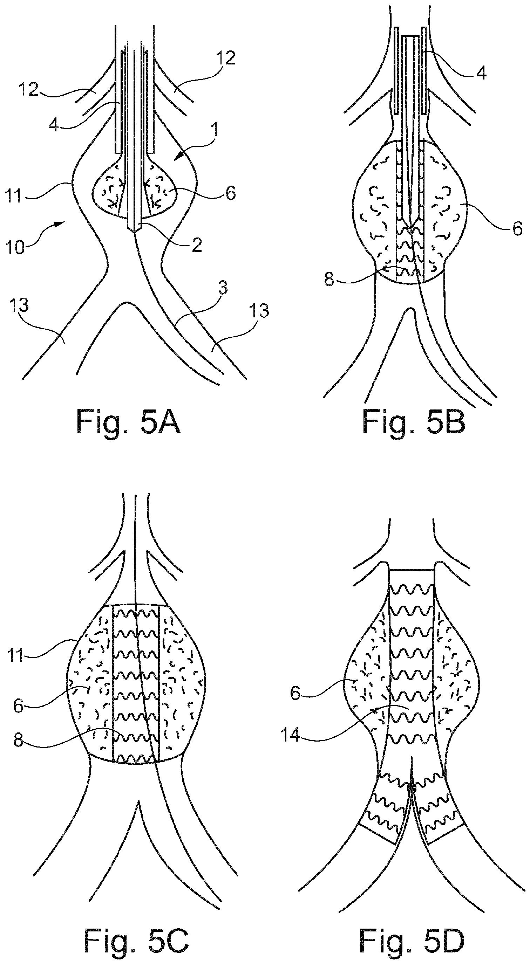

Figures 5A - 5D show the abdominal aorta 10 with an abdominal aortic aneurysm 11, renal arteries 12 and common iliac arteries 13.

The catheter system 1 is introduced into the abdominal aorta 10 in a known way. The filling body 6 is pushed out of the internal volume 5, such that the filling body 6 expands into the abdominal aortic aneurysm 11. (see figure 5A en 5B). Upon expansion, the nitinol stent 8 also expands (see figure 5B) such that a cylindrical lumen is provided in the filling body 6. (see figure 5C)

The cylindrical lumen formed by the nitinol stent 8 allows for a passthrough opening through the abdominal aortic aneurysm 11, which is now completely filled with the porous filling body 6.

This passthrough opening can now be used to position a common prosthesis 14 into the abdominal aorta 10.

## Claims

1. Catheter system (1) for delivery of a filling body (6) to an aneurysmal sac (11) in a body lumen, which catheter system (1) comprises:
- a substantially tubular shaft (2) having a proximal end, a distal end and a guidewire lumen extending between the proximal and distal ends;
- a substantially tubular sheath (4) defining an interior volume (5), coaxially positioned over the tubular shaft (2) and slidable relative to the tubular shaft (2);
- a compressible filling body (6) arranged in a compressed state in the interior volume (5) between the sheath (4) and the shaft (2) near the distal end of the tubular shaft (2) ;
wherein the compressible filling body (6) is expandable, when released from the sheath (4), to a filling body (6) having a substantially cylindrical lumen (8) and an outer surface (7) having a main dimension (D₂) perpendicular to the longitudinal axis of the cylindrical lumen (8), which main dimension (D₂) is at least 1,5 times the diameter of the cylindrical lumen (D₁),
**characterized in that**
the expanded filling body (6) is porous and the material of the filling body (6) has an open cell structure, preferably of a shape memory polymer or other memory material.

2. Catheter system (1) according to claim 1, wherein the outer surface (7) is non-cylindrical, preferably bulbous shaped.

3. Catheter system (1) according to claim 1 or 2, wherein a nitinol stent (9) is arranged in the cylindrical lumen (8) of the filling body (6).

4. Catheter system (1) according to claim 1, wherein the material of the filling body (6) is a non-isotropic 3D printed foam with a structure similar to cancellous and cortical fibres.

5. Catheter system (1) according to any of the preceding claims, wherein the diameter of the circumscribing circle of the non-cylindrical outer surface (7) in a plane perpendicular to the longitudinal axis of the substantially cylindrical lumen (8) is between 2cm and 10cm, preferably between 5cm and 10cm.

6. Catheter system (1) according to any of the preceding claims, wherein the filling body (6) is coated with a hemostatic coating, such as fibrinogen, fibrin or collagen.

## Patentansprüche

1. Kathetersystem (1) zur Zufuhr eines Füllkörpers (6) zu einem Aneurysmasack (11) in einem Körperlumen, wobei das Kathetersystem (1) Folgendes umfasst:
- einen im Wesentlichen röhrenförmigen Schaft (2), der ein proximales Ende, ein distales Ende und ein Führungsdrahtlumen aufweist, das sich zwischen dem proximalen und dem distalen Ende erstreckt;
- eine im Wesentlichen röhrenförmige Hülle (4), die ein Innenvolumen (5) definiert, koaxial über dem röhrenförmigen Schaft (2) positioniert und relativ zum röhrenförmigen Schaft (2) verschiebbar ist;
- einen komprimierbaren Füllkörper (6), der in komprimiertem Zustand im Innenvolumen (5) zwischen der Hülle (4) und dem Schaft (2) nahe dem distalen Ende des rohrförmigen Schafts (2) angeordnet ist;
wobei der komprimierbare Füllkörper (6) beim Freigeben aus der Hülle (4) zu einem Füllkörper (6) expandierbar ist, der ein im Wesentlichen zylindrisches Lumen (8) und eine Außenfläche (7) aufweist, die eine Hauptabmessung (D₂) senkrecht zur Längsachse des zylindrischen Lumens (8) aufweist, wobei die Hauptabmessung (D₂) mindestens das 1,5-fache des Durchmessers des zylindrischen Lumens (D₁) beträgt,
**dadurch gekennzeichnet, dass**
der expandierte Füllkörper (6) porös ist und das Material des Füllkörpers (6) eine offenzellige Struktur aufweist, bevorzugt aus einem Formgedächtnispolymer oder einem anderen Gedächtnismaterial.

2. Kathetersystem (1) nach Anspruch 1, wobei die Außenfläche (7) nicht zylindrisch, bevorzugt bauchig geformt ist.

3. Kathetersystem (1) nach Anspruch 1 oder 2, wobei im zylindrischen Lumen (8) des Füllkörpers (6) ein Nitinol-Stent (9) angeordnet ist.

4. Kathetersystem (1) nach Anspruch 1, wobei das Material des Füllkörpers (6) ein nicht-isotroper 3D-gedruckter Schaum mit einer Struktur ist, die spongiösen und kortikalen Fasern ähnelt.

5. Kathetersystem (1) nach einem der vorstehenden Ansprüche, wobei der Durchmesser des umschreibenden Kreises der nicht-zylindrischen Außenfläche (7) in einer Ebene senkrecht zur Längsachse des im Wesentlichen zylindrischen Lumens (8) zwischen 2 cm und 10 cm, bevorzugt zwischen 5 cm und 10 cm beträgt.

6. Kathetersystem (1) nach einem der vorstehenden Ansprüche, wobei der Füllkörper (6) mit einer blutstillenden Beschichtung, beispielsweise Fibrinogen, Fibrin oder Kollagen, beschichtet ist.

## Revendications

1. Système de cathéter (1) pour l'administration d'un corps de remplissage (6) dans un sac anévrismal (11) dans une lumière corporelle, lequel système de cathéter (1) comprend :
- une tige sensiblement tubulaire (2) présentant une extrémité proximale, une extrémité distale et une lumière de fil-guide s'étendant entre les extrémités proximale et distale ;
- une gaine sensiblement tubulaire (4) définissant un volume intérieur (5), positionnée coaxialement sur la tige tubulaire (2) et pouvant coulisser par rapport à la tige tubulaire (2) ;
- un corps de remplissage compressible (6) agencé dans un état comprimé dans le volume intérieur (5) entre la gaine (4) et la tige (2) à proximité de l'extrémité distale de la tige tubulaire (2) ;
dans lequel le corps de remplissage compressible (6) est dilatable, lorsqu'il est libéré de la gaine (4), jusqu'à devenir un corps de remplissage (6) présentant une lumière sensiblement cylindrique (8) et une surface externe (7) présentant une dimension principale (D₂) perpendiculaire à l'axe longitudinal de la lumière cylindrique (8), dont la dimension principale (D₂) correspond à au moins 1,5 fois le diamètre de la lumière cylindrique (D₁),
**caractérisé en ce que**
le corps de remplissage dilaté (6) est poreux et le matériau du corps de remplissage (6) présente une structure cellulaire ouverte, de préférence un polymère à mémoire de forme ou un autre matériau à mémoire de forme.

2. Système de cathéter (1) selon la revendication 1, dans lequel la surface externe (7) est de forme non cylindrique, de préférence bulbeuse.

3. Système de cathéter (1) selon la revendication 1 ou 2, dans lequel un stent en nitinol (9) est agencé dans la lumière cylindrique (8) du corps de remplissage (6).

4. Système de cathéter (1) selon la revendication 1, dans lequel le matériau du corps de remplissage (6) est une mousse non isotrope imprimée en 3D avec une structure similaire aux fibres spongieuses et corticales.

5. Système de cathéter (1) selon l'une quelconque des revendications précédentes, dans lequel le diamètre du cercle circonscrit de la surface externe non cylindrique (7) dans un plan perpendiculaire à l'axe longitudinal de la lumière sensiblement cylindrique (8) est compris entre 2 cm et 10 cm, de préférence entre 5 cm et 10 cm.

6. Système de cathéter (1) selon l'une quelconque des revendications précédentes, dans lequel le corps de remplissage (6) est recouvert d'un revêtement hémostatique, tel que du fibrinogène, de la fibrine ou du collagène.
